# EUROPEAN PATENT APPLICATION

(11) **EP 1 842 848 A1**
(43) Date of publication of application: **10.10.2007**
(21) Application number: 06290532.8
(22) Date of filing: 03.04.2006
(51) Int. Cl.: C07D 265/30, C07C 47/228, C07C 22/04

(54) **Process for producing 3-[4-(1,1-dimethyl-propyl)-phenyl]2-methyl-propionaldehyde and cis-4{3-[4-(1,1-dimethyl-propyl)-phenyl]2-methyl-propyl}-2,6-dimethyl-morpholine (amorolfine)**

(71) Applicant: Galderma S.A., 6330 Cham (CH)
(72) Inventor: Boiteau, Jean-Guy, 34130 Saint-Aunes (FR); Musicki, Branislav, 06000 Nice (FR)
(74) Representative: Allab, Myriam

(57) **Abstract**

The present invention discloses a new process for the preparation of 3-[4-(1,1-dimethyl-propyl)-phenyl]-2-methyl-propionaldehyde and for the preparation of *cis*-4-{3-[4-(1,1-dimethyl-propyl)-phenyl]-2-methyl-propyl}-2,6-dimethyl-morpholine (Amorolfine).

## Description

The present invention relates to a new process for the synthesis of 3-[4-(1,1-dimethylpropyl)-phenyl]-2-methyl-propionaldehyde and *cis*-4-{3-[4-(1,1-dimethyl-propyl)-phenyl]-2-methyl-propyl}-2,6-dimethyl-morpholine or its salts.

Amorolfine, *cis*-4-{3-[4-(1,1-dimethyl-propyl)-phenyl]-2-methyl-propyl-2,6-dimethylmorpholine of formula (I) is a potent antifungal drug exhibiting a large spectrum of in vitro activity. It belongs to a new chemical class of antimycotics and exhibits a broad spectrum of antifungal activity against dermatophytes, dimorphic fungi, Candida albicans, Cryptococus neoformans and some dematiaceae. It finds a major use as the active ingredient in nail lacquer as a topical antifungal in treatment of onychomycosis and as topical antimycotic indicated for the treatment of dermatomycosis.

Zhixiang teaches a multi-step synthesis of Amorolfine from tert-pentylbenzene (F. Zhixiang & coll., Zhongguo Yaowu Huaxue Zazhi, 10(1), 64-65, (2000)). By hydroxymethylation and bromination from tert-pentylbenzene, 4-bromomethyl-tert-pentylbenzene is prepared. It is coupled with methyl malonic ester, hydrolyzed and decarboxylated to afford 2-methyl-3-(4-tert-pentylphenyl) propionic acid. This intermediate is amidated with *cis*-2,6-dimethylmorpholine and reduced to afford Amorolfine of formula (I).

Hoffmann-La Roche patent (FR 2,463,767 or EP 24,334) disclose other synthetic pathways for the production of Amorolfine of formula (I) or its hydrochloride salt of formula (Ia). In the Hoffmann-La Roche patent, Amorolfine is produced as a racemic mixture of the *cis* isomers by tree different synthetic pathways.

A first synthetic pathway discloses a *nucleophilic substitution* on 1-(3-halo-2-methylpropyl)-4-(1,1-dimethyl-propyl)-benzene (halo=chlorine, bromine or iodine) by cis-2,6-dimethyl morpholine to produce Amorolfine of formula (I)

A second synthetic pathway discloses a *reduction* of a compound of the formula 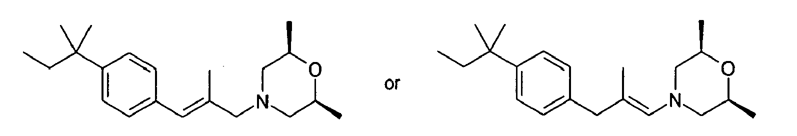 to produce Amorolfine of formula (I)

A third synthetic pathway discloses an *amino reduction* between (E)-2-Methyl-3-phenyl-propenal and cis-2,6-dimethyl morpholine to produce cis-2,6-dimethyl-4-(2-methyl-3-phenyl-propyl)-morpholine followed by a *Friedel-Craft* reaction with 2-methyl-2-butanol to produce Amorolfine of formula (I)

On another side, Fenpropimorph, 4-[3-(4-tert-butylphenyl)-2-methylpropyl]-2,6-dimethylmorpholine, which is a pesticide, specifically categorised as a morpholine fungicide, has been recently synthesised by Forsyth in a two steps one pot process (S.A.Forsyth & coll; Organic Process Research & Development, 10, 94-102, (2006)).

This process teaches a *Heck* reaction between 4-*tert*-butyl-iodobenzene and 2-methyl-prop-2-en-1-ol catalyzed by palladium chloride (PdCl₂) in ionic liquid solvent followed by an *amino reduction* in presence of cis-2,6-dimethyl morpholine catalyzed by palladium on carbon (Pd/C) under hydrogen pressure in ionic liquid solvent.

We have now found that Amorolfine or its salts can be produced in a two steps one pot process involving a *Heck* reaction between (1,1-dimethyl-propyl)-4-iodo-benzene and 2-methyl-prop-2-en-1-ol catalyzed by palladium catalyst such as palladium acetate (PdOAc₂) in N,N-dimethylformamide (DMF) followed by an *amino reduction* in alcohol in presence of *cis*-2,6-dimethyl morpholine and a reducing agent such as hydrogen gas/palladium catalyst or a mixed metal hydrides, in alcoholic solvents.

This new process has never been used in the prior art to synthesize Amorolfine.

The solvent used in the invention for the *Heck* reaction is DMF, polar protic or non polar organic solvents.

Likewise, the solvent used in the invention for the *amino reduction* reaction is alcohol.

With such a two steps one pot process, the production of Amorolfine is optimized.

This invention is a new process for preparing 3-[4-(1,1-dimethyl-propyl)-phenyl]-2-methyl-propionaldehyde of formula (II): 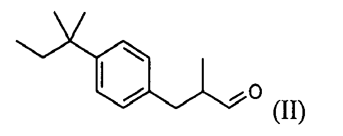 characterized in that a compound of general formula (VI) 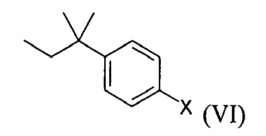 in which X can be an halide such as bromine, chlorine, iodine or fluorine, or a trifluoromethane sulfonate radical (OSO₂CF₃) is reacting in Heck reaction with 2-methyl-prop-2-en-1-ol of formula (VII) 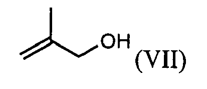 in the presence of palladium catalyst and a base in a solvent chosen from N,N-dimethylformamide (DMF), polar protic and non polar organic solvents, at temperature between 60° to 150°C

This invention extends to a new process of producing Amorolfine of formula (I) 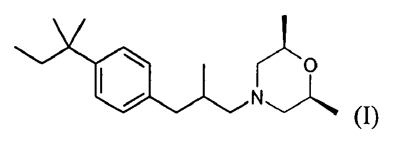 or its salts characterized in that in the first step, compound of general formula (VI) 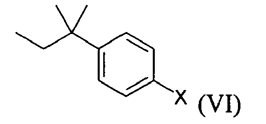 in which X is an halide such as bromine, chlorine, iodine or fluorine or a trifluoromethane sulfonate radical (OSO₂CF₃) is subjected to Heck coupling conditions with 2-methyl-prop-2-en-1-ol of formula (VII) 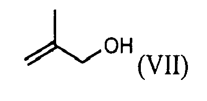 in the presence of palladium catalyst and a base in a solvent chosen from N,N-dimethylformamide (DMF), polar protic and non polar organic solvents, at temperature between 60° and 150°C, to provide 3-[4-(1,1-dimethyl-propyl)-phenyl]-2-methyl-propionaldehyde of formula (II): 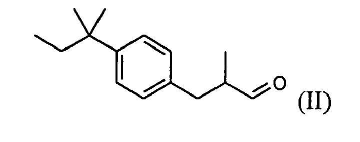 and in the second step (amino reduction step), 3-[4-(1,1-dimethyl-propyl)-phenyl]-2-methyl-propionaldehyde of formula (II) is reacted with cis-2,6-dimethyl morpholine of formula (IV) 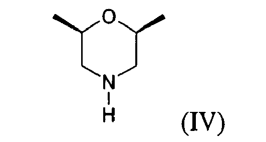 in the presence of a reducing agent in a solvent to furnish *cis*-4-{3-[4-(1,1-dimethyl-propyl)-phenyl]-2-methyl-propyl}-2,6-dimethyl-morpholine of formula (I) (Amorolfine):

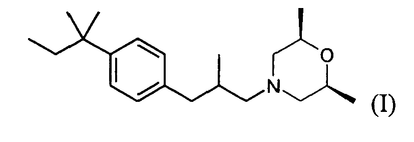

This compound can be further converted to a corresponding salt of *cis-*4-{3-[4-(1,1-dimethylpropyl)-phenyl]-2-methyl-propyl}-2,6-dimethyl-morpholine. The salification can be performed thanks to the addition of an acid, like hydrochloric acid.

The two steps of the present invention as described above can be realized advantageously in a "one pot" process without isolation of the aldehyde intermediate of formula (II).

The preferred salt of *cis*-4-{3-[4-(1,1-dimethyl-propyl)-phenyl]-2-methyl-propyl}-2,6-dimethyl-morpholine is the hydrochloride salt of formula (Ia)

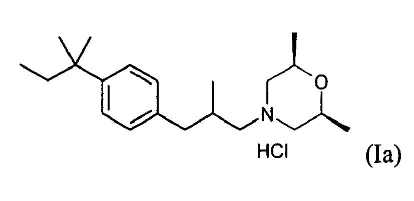

Particularly, the palladium catalyst used in the Heck reaction conditions of the invention is chosen among palladium(II)acetate, palladium(II)chloride, tetrakis (triphenylphosphine)-palladium(0), palladium on activated carbon, dichloro [1,1'-bis(diphenylphosphino)ferrocene] palladium(II), dichloro-bis-triphenylphosphino palladium(II).

Notably, a phosphinic ligand can be used in combination with the palladium catalyst. Commonly phosphinic ligands used are for example triphenylphosphine, tri-o-tolylphosphine, tri-m-tolylphosphine or tri-p-tolylphosphine.

In this first step, the organic solvents used, according to the invention, are *N,N-*dimethylformamide (DMF), polar protic solvent as for example methanol, ethanol, n-propanol, i-propanol, n-butanol, i-butanol, t-butanol or appropriate mixture of these solvents with water or non polar solvent as for exemple tetrahydrofurane, ethyl acetate, toluene, o-xylene, m- xylene, p-xylene.

The reaction is carried out in the presence of a base such as a tertiary amine, as for example triethylamine, tripropylamine, tributylamine, diisopropylethylamine, a metal carbonates, as for example sodium carbonate, sodium bicarbonate, potassium carbonate, potassium bicarbonate or a metal acetates as for example potassium acetate or sodium acetate.

Advantageously, the reaction is carried out in an inert atmosphere, such as under nitrogen or argon gas in a suitable reaction vessel.

The temperatures are hold between about 60° to about 150°C during about 30 min to 24 h.

Advantageously, the molar ratio between the two starting materials (VI)/(VII) range from (VI)/(VII) =1/1 to (VI)/(VII) =1/2.

In the field of the invention, the preferred conditions for the Heck reaction are DMF as solvent, an homogeneous palladium catalyst such as palladium(II) acetate, a base such as sodium carbonate, a range time between 8 hours and 10 hours, for example 9 hours, and a temperature between 80° and 110°C.

As starting material, the preferred compound of general formula (VI) is the (1,1-dimethyl-propyl)-4-iodo-benzene of formula (VIa)

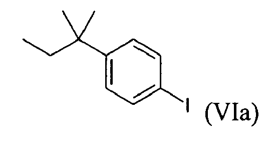

Only one synthetic method is reported in the literature for this starting material. Unfortunately, the used conditions (HI-I₂) gave only a moderate 60% yield starting from (1,1-dimethyl-propyl)-benzene of formula (VIII) (E. Boedtker, Bull. Soc. Chim., 45, 645-650, (1929)).

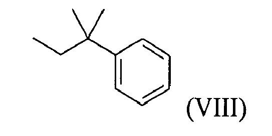

A new process for iodination reaction of compound of formula (VIII) is achieved by using sodium metaperiodate and iodine in a mixture of acetic acid and acetic anhydride followed by the addition of sulfuric acid. In those conditions, (1,1-dimethyl-propyl)-4-iodobenzene of formula (VIa) is obtained in excellent yield (98%)

The amino reduction step is performed with reducing agent such as :
- hydrogen gas in the presence of a palladium catalyst such as for example palladium on activated carbon, palladium on activated carbon in the presence of metal salts like Ba(OH)₂, Ca(OH)₂, CaCO₃, BaCO₃ or Perlmans catalyst Pd(OH)₂.
- or mixed metal hydrides like metal borohydride such as for example sodium borohydride (NaBH₄) and lithium borohydride (LiBH₄), or like metal cyano borohydride such as for example sodium cyano borohydride (NaCNBH₃) and lithium cyanoborohydride (LiCNBH₃).

The solvent in which the amino reduction is performed is N,N-dimethylformamide, a polar protic solvent such as methanol, ethanol, propanol, i-propanol, butanol, iso-butanol, tert-butanol or a non polar organic solvent such as toluene, tetrahydrofurane, ethyl acetate.

The temperature for this amino reduction may typically be set at no more than 45°C, preferably between 20° and 45°C, and more preferably between 20° and 30°C in the case of metal catalyst such as palladium catalyst, and preferably between 0°C and 30°C in the case of mixed metal hydride.

When the two steps of the present invention as described above are realized advantageously in a "one pot" process without isolation of the aldehyde intermediate of formula (II), the preferred conditions are to
a) first react (1,1-dimethyl-propyl)-4-iodo-benzene of formula (VIa) with 2-methyl-prop-2-en-1-ol of formula (VII) in DMF as solvent and in the presence of palladium acetate (PdOAc₂) as catalyst and sodium bicarbonate (NaHCO₃) as base, at 100°C during 9h.
b) filtrate the catalyst through Celite, then add ethanol, acetic acid and *cis*-2,6-dimethylmorpholine of formula (IV).
c) Cool the mixture to -5°C, then add sodium borohydride (NaBH₄)
d) After work-up, dissolve the crude product obtained from step c) in diisopropyl oxide (iPr₂O) and add a solution of hydrochloric acid gas (HCl) in ethyl acetate (EtOAc)
e) isolate cis-4-{3-[4-(1,1-dimethyl-propyl)-phenyl]-2-methyl-propyl}-2,6-dimethyl-morpholine hydrochloride of formula (Ia) obtained by filtration.

The present invention will now be described by way of example. The following examples are not intended to be limiting on the invention.

### Example 1: Two steps synthesis of cis-4-{3-[4-(1,1-dimethyl-propyl)-phenyl]-2-methylpropyl}-2,6-dimethyl-morpholine hydrochloride (Ia) using NaBH₄ as reducing agent

### First step:

### a) (1,1-dimethyl-propyl)-4-iodo-benzene (VIa)

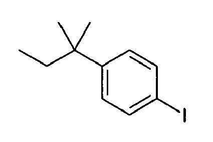

To a mixture of AcOH (1188 mL) and Ac₂O (594 mL) was added sodium periodate (79.83 g, 0.373 mol,) and iodine (280.94 g, 1.107 mol,). Then the mixture was cooled in icebath and concentrated. sulfuric acid (184 mL, 3.71 mol) was added drop-wise during 20 min so that temperature did not exceed 12°C. After the addition was complete, (1,1-dimethylpropyl)-benzene (256.76 g, 1.732 mol, 1.0 eq)) was added at once and the stirring continued for 24 h. The reaction was then partitioned between heptane-10% EtOAc (1L) and water (2L). The organic phase was separated and was washed with water (2L) containing Na₂SO₃ (50 g). Then the organic phase was dried over MgSO₄ and the solvents were evaporated.

457.87 g of (1,1-dimethyl-propyl)-4-iodo-benzene were isolated as a liquid (96% yield). This material was pure enough to be used in the next step without purification.
¹H NMR (400 MHz, CDCl₃) δ: 0.73 (3H, t, J = 7.4 Hz), 1.31 (6H, s), 1.67 (2H, q, J = 7.4 Hz), 7.13 (2H, d, J = 8.56 Hz), 7.66 (2H, d, J = 8.56 Hz);
¹³C NMR (100 MHz, CDCl₃) δ: 9.59 (CH₃), 28.78 (2CH3), 37.16 (CH2), 38.3 (C_{A1}), 91.12 (C_{Ar}), 128.72 (CH_{Ar}), 137.46 (CH_{Ar}), 149.62 (C_{Ar}).

### b) 3-[4-(1,1-Dimethyl-propyl)-phenyl]-2-methyl-propionaldehyde (II)

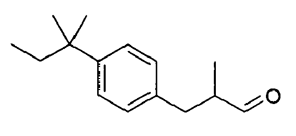

Through a mixture of (1,1-dimethyl-propyl)-4-iodo-benzene (137.07 g, 0.50 mol), palladium(II) acetat (1.122 g, 5 mmol) and sodium bicarbonate (50.40 g, 0.60 mol) in dry DMF (500 mL) was bubbled nitrogen for 10 min. Then 2-methyl-prop-2-en-1-ol (54.083 g, 0.75 mol) was added and nitrogen was bubbled for another ten minutes. The reaction mixture was heated under nitrogen for 9 h at 100°C. The reaction was cooled and was filtered through a thin layer of Celite. The Celite was then washed with DMF (300 mL). The reaction was then partitioned between H₂O (2 L) and heptane/EtOAc (9/1) (2x1 L). The combined organic layers were washed with H₂O (1 L) and were dried over MgSO₄. The solvents were evaporated.
109.63 g of 3-[4-(1,1-dimethyl-propyl)-phenyl]-2-methyl-propionaldehyde were isolated as crude product. This intermediate was purified by distillation at 112°C under 0.06 mbar to afford 84 g of pure 3-[4-(1,1-dimethyl-propyl)-phenyl]-2-methyl-propionaldehyde (77% yield)..
¹H NMR (400 MHz, CDCl₃) δ: 0.69 (3H, t, J=7.45), 1.11 (3H, d, J=6.87), 1.29 (6H, s), 1.65 (2H, q, J=7.43), 2.60 (1H_{AB}, dd, *J=* 8.18, 13.52 Hz), 2.69 (1H, sex, J=7.06), 3.08 (1H, dd, *J =* 5.87, 13.54 Hz), 7.12 (2H, d, J = 8.27), 7.27 (2H, d, J = 8.27), 9.75 (1H, s);
¹³C NMR (100 MHz, CDCl₃) δ: 9.6 (CH₃), 13.7 (CH₃), 25.8 (CH₃), 36.6 (CH₂), 37.3 (CH₂), 38.0 (C), 48.5 (CH), 126.4 (CH_{Ar}), 128.6 (CH_{Ar}), 136.0 (C_{Ar}), 148.0 (C_{Ar}), 205.1 (C=O).

### Second step:

### c) cis-4-{3-[4-(1,1-Dimethyl-propyl)-phenyl]-2-methyl-propyl}-2,6-dimethylmorpholine hydrochloride (Ia)

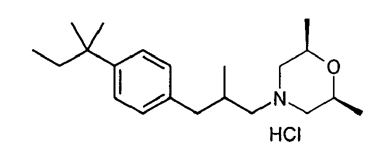

3-[4-(1,1-dimethyl-propyl)-phenyl]-2-methyl-propionaldehyde (109.63 g ; 0.50 mol) obtained at the previous step were dissolved in absolute ethanol (500 mL). To the ethanol solution was added at 0°C (ice/water) AcOH (30 mL, 0.5 mol) and cis-2,6-dimethylmorpholine (69.108 g, 0.60 mol) and the reaction was stirred at RT for 30 min. It was then cooled to (-15°C) and NaBH₄ (15.93 g, 0.42 mol) was added in portions during 1 h. Stirring was continued at 0°C for 2 h and the reaction was quenched by drop-wise addition of NaOH solution in water (30 g, 0.75 mol, /100 mL H₂O) to pH=12. The reaction was then partitioned between H₂O (2 L) and hept/EtOAc (9/1) (2x1 L). The organic phases were combined, washed with the H₂O (1L), and dried over MgSO4. After evaporation of the solvents, the residue (147.14 g) was dissolved in iPr₂O (500 mL) and cooled to 0°C. To this solution was added the solution of HCl gas in EtOAc (150 ml, ~4M) drop-wise (30 min) and with stirring. White precipitate that was formed during HCl addition was filtered 30 min after completion of the addition. It was washed with iPr₂O (300 mL) and was dried in vacuo at 40°C during 48 h providing 118.79 g of cis-4-{3-[4-(1,1-dimethyl-propyl)-phenyl]-2-methyl-propyl}-2,6-dimethyl-morpholine hydrochloride as white powder. (67% yield) (Melting Point = 205.9°C)
¹H NMR (400 MHz, CDCl₃) δ: 0.63 (3H, t, J = 7.39 Hz), 1.12 (3H, d, J = 6.30 Hz), 1.13 (3H, d, J = 6.32 Hz), 1.24 (6H, s), 1.26 (3H, d, J = 6.65 Hz), 1.59 (2H, q, J = 7.49 Hz), 2.05 (1H, q, J = 10.77 Hz), 2.26 (1H, q, J = 10.80 Hz), 2.27-2.35 (1H, m), 2.56-2.66 (2H, m), 2.76-2.89 (2H, m), 3.22 (2H, t, J = 13.28 Hz), 4.36-4.44 (1H, m), 4.46-4.54 (1H, m), 7.06 (2H, d, J = 8.22 Hz), 7.23 (2H, d, J = 8.22 Hz), 12.55 (1H, b)

### Example 2: One pot synthesis of cis-4-{3-[4-(1,1-dimethyl-propyl)-phenyl]-2-methylpropyl}-2,6-dimethyl-morpholine hydrochloride (Ia) using NaBH₄ as reducing agent

Through a mixture of (1,1-dimethyl-propyl)-4-iodo-benzene (137.07 g, 050 mol), palladium(II) acetat (1.122 g, 5 mmol) and sodium bicarbonate (50.40 g, 0.60 mol) in dry DMF (500 mL) was bubbled nitrogen for 10 min. Then 2-methyl-prop-2-en-1-ol (54.083 g, 0.75 mol) was added and nitrogen bubbled for another ten minutes. The reaction mixture was heated under nitrogen for 9 h at 100°C. The reaction was cooled (0°C)and was filtered through a thin layer of Celite. The Celite was then washed with cold DMF (300 mL). To DMF solution was added EtOH (abs. 700 mL) followed by AcOH (30 mL) and *cis-2,6-*dimethylmorpholine (73.9 mL). After 15 min stirring at RT, the reaction was cooled to -5°C and NaBH₄ (15.93 g, 0.42 mol) was added in portions over 1h 30 min so that temperature did not exceed 2-3°C. Stirring was continued at 0°C for 1 h and the reaction was quenched by drop-wise addition of NaOH solution in water at 0°C (30 g, 0.75 mol, /100 mL H₂O) to pH=12. The reaction was then partitioned between H₂O (2 L) and Hept/EtOAc (9/1) (2x1 L). The organic phases were combined, washed with the H₂O (1L), and dried over MgSO₄. After evaporation of the solvents, the residue (147.14 g) was dissolved in iPr₂O (500 mL) and cooled to 0°C. To this solution was added the solution of HCl gas in EtOAc (150 ml, ~4M) drop-wise (30 min) and with stirring. White precipitate was formed during HCl addition and was filtered 30 min after completion of the addition. It was washed with iPr₂O (300 mL) and was dried in vacuo at 40°C during 48 h providing 112.34 g of cis-4-{3-[4-(1,1-dimethylpropyl)-phenyl]-2-methyl-propyl}-2,6-dimethyl-morpholine hydrochloride as white powder. (64% yield) (Melting Point = 205.8°C)
¹H NMR (400 MHz, CDCl₃) δ: 0.63 (3H, t, J = 7.39 Hz), 1.12 (3H, d, J = 6.30 Hz), 1.13 (3H, d, J = 6.32 Hz), 1.24 (6H, s), 1.26 (3H, d, J = 6.65 Hz), 1.59 (2H, q, J = 7.49 Hz), 2.05 (1H, q, J = 10.77 Hz), 2.26 (1H, q, J = 10.80 Hz), 2.27-2.35 (1H, m), 2.56-2.66 (2H, m), 2.76-2.89 (2H, m), 3.22 (2H, t, J = 13.28 Hz), 4.36-4.44 (1H, m), 4.46-4.54 (1H, m), 7.06 (2H, d, J = 8.22 Hz), 7.23 (2H, d, J = 8.22 Hz), 12.55 (1H, b)

### Example 3: Two steps synthesis of cis-4-{3-[4-(1,1-dimethyl-propyl)-phenyl]-2-methylpropyl}-2,6-dimethyl-morpholine hydrochloride (Ia) using hydrogen and palladium on carbon as reducing agent

### First step:

Same as example 1

### Second step:

3-[4-(1,1-dimethyl-propyl)-phenyl]-2-methyl-propionaldehyde (100.56 g, 0,46 mol) obtained as described in example 1-b) was dissolved in absolute methanol (500 mL). To the methanol solution cooled at 0°C (ice/water) was added AcOH (30 mL, 0.50 mol) and *cis-2,6-*dimethylmorpholine (69.108, 0.60 mol) and the reaction was stirred at room temperature for 30 min. It was then placed under nitrogen and Pd/C 10% (5.00 g) added to it. nitrogen was replaced with hydrogen and the reaction was heated at 37°C under hydrogen pressure. At the end of the reduction. the reaction mixture was filtered through Celite. The Celite was washed with MeOH (200 mL), then aqueous NaOH solution was added to the methanolic reaction mixture until pH=12. The reaction mixture was partitioned with Hept/EtOAc (9/1) (2x1 L), the organic phase was washed with water (1 L) and was dried over MgSO₄. After evaporation of the solvents, the residue (144.32 g) was dissolved in iPr₂O (500 mL) and cooled to 0°C. To this solution was added a solution of HCl gas in EtOAc (150 ml, ~4M) drop-wise (30 min) and with stirring. White precipitate formed during HCl in EtOAc addition was filtered 30 min after completion of the addition. It was washed with iPr₂O (300 mL) and was dried in vacuo at 40°C during 48 h providing 112.65 g of cis-4-{3-[4-(1,1-dimethyl-propyl)-phenyl]-2-methyl-propyl}-2,6-dimethyl-morpholine hydrochloride as white powder. (64% yield) (Melting Point = 205.7°C)
¹H NMR (400 MHz, CDCl₃) δ: 0.63 (3H, t, J = 7.39 Hz), 1.12 (3H, d, J = 6.30 Hz), 1.13 (3H, d, J = 6.32 Hz), 1.24 (6H, s), 1.26 (3H, d, J = 6.65 Hz), 1.59 (2H, q, J = 7.49 Hz), 2.05 (1H, q, J = 10.77 Hz), 2.26 (1H, q, J = 10.80 Hz), 2.27-2.35 (1H, m), 2.56-2.66 (2H, m), 2.76-2.89 (2H, m), 3.22 (2H, t, J = 13.28 Hz), 4.36-4.44 (1H, m), 4.46-4.54 (1H, m), 7.06 (2H, d, J = 8.22 Hz), 7.23 (2H, d, J = 8.22 Hz), 12.55 (1H, b)

## Claims

1. Process for preparing 3-[4-(1,1-dimethyl-propyl)-phenyl]-2-methyl-propionaldehyde of formula (II): 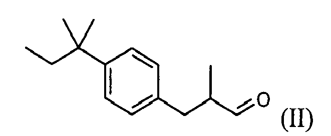 **characterized in that** a compound of general formula (VI) 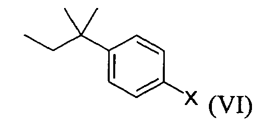 in which X is an halide or a trifluoromethane sulfonate radical (OSO₂CF₃) is reacting in Heck reaction with 2-methyl-prop-2-en-1-ol of formula (VII) 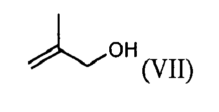 in the presence of palladium catalyst and a base in a solvent chosen from N,N-dimethylformamide, polar protic and non polar organic solvents at temperature between 60° to 150°C.

2. Process of producing Amorolfine of formula (I) 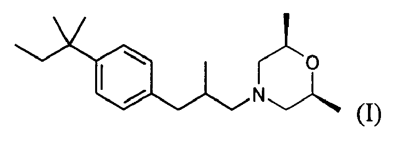 or its salts, **characterized in that** it comprises, after the process according to claim 1, an amino reduction step consisting in reacting 3-[4-(1,1-dimethyl-propyl)-phenyl]-2-methyl-propionaldehyde of formula (II) with *cis*-2,6-dimethyl morpholine of formula (IV) 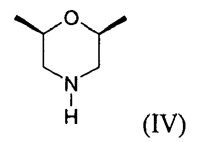 in the presence of a reducing agent in a solvent.

3. Process as claimed in claim 2, wherein Amorolfine is converted to a corresponding salt by addition of an acid.

4. Process as claimed in one of claims 1 to 3 **characterized in that** the palladium catalyst used in the Heck reaction is selected from the group consisting of palladium(II)acetate, palladium(II)chloride, tetrakis (triphenylphosphine)-palladium(0), palladium on activated carbon, dichloro [1,1'-bis(diphenylphosphino)ferrocene] palladium(II) and dichloro-bis-triphenylphosphino palladium(II).

5. Process as claimed in one of claims 1 to 4 **characterized in that** a phosphinic ligand is used in combination with the palladium catalyst.

6. Process as claimed in claim 5 **characterized in that** the phosphinic ligand is selected from the group consisting of triphenylphosphine, tri-o-tolylphosphine, tri-m-tolylphosphine and tri-p-tolylphosphine

7. Process as claimed in one of claims 1 to 6 **characterized in that** the polar protic solvent used in the Heck reaction is selected from the group consisting of methanol, ethanol, n-propanol, i-propanol, n-butanol, i-butanol, t-butanol and appropriate mixture of these solvents with water.

8. Process as claimed in one of claims 1 to 7 **characterized in that** the non polar organic solvent used in the Heck reaction is selected from the group consisting of tetrahydrofurane, ethyl acetate, toluene, o- xylene, m- xylene and p-xylene.

9. Process as claimed in one of claims 1 to 8 **characterized in that** the base used in the Heck reaction is selected from the group consisting of a tertiary amine, a metal carbonate, and a metal acetate.

10. Process as claimed in one of claims 2 to 9 **characterized in that** the reducing agent used in the amino reduction step is selected from the group consisting of hydrogen gas in the presence of a palladium catalyst and mixed metal hydrides.

11. Process as claimed in claim 10 **characterized in that** the palladium catalyst used in the amino reduction step is selected from the group consisting of palladium on activated carbon, palladium on activated carbon in the presence of metal salts or Perlmans catalyst.

12. Process as claimed in claim 10 **characterized in that** the mixed metal hydride used in the amino reduction step is selected from the group consisting of sodium borohydride, lithium borohydride, sodium cyano borohydride and lithium cyanoborohydride.

13. Process as claimed in one of claims 2 to 12 **characterized in that** the polar protic solvent used in the amino reduction step is selected from the group consisting of methanol, ethanol, propanol, i-propanol, butanol, iso-butanol and tert-butanol.

14. Process as claimed in one of claims 2 to 13 **characterized in that** the non polar solvent used in the amino reduction step is selected from the group consisting of toluene, tetrahydrofurane and ethyl acetate.

15. Process as claimed in claim 11 **characterized in that** the temperature in the amino reduction step is no more than 45°C, preferably between 20° and 45°C, and more preferably between 20° and 30°C.

16. Process as claimed in claim 12 **characterized in that** the temperature in the amino reduction step is between 0°C and 30°C.

17. Process as claimed in one of claims 2 to 16 **characterized in that** the two steps are performed in a one pot process without isolation of the aldehyde intermediate of formula (II).

18. Process as claimed in claim 17 **characterized in that** it comprises the following steps:
a) first react (1,1-dimethyl-propyl)-4-iodo-benzene with 2-methyl-prop-2-en-1-ol in N,N-dimethylformamide and in the presence of palladium acetate and sodium bicarbonate, at 100°C during 9h;
b) filtrate the catalyst through Celite, then add ethanol, acetic acid and *cis-*2,6-dimethylmorpholine;
c) Cool the mixture to -5°C, then add sodium borohydride ;
d) After work-up, dissolve the crude product obtained from step c) in diisopropyl oxide and add a solution of hydrochloric acid gas in ethyl acetate;
e) isolate cis-4-{3-[4-(1,1-dimethyl-propyl)-phenyl]-2-methyl-propyl}-2,6-dimethyl-morpholine hydrochloride by filtration.

19. Process for preparing (1,1-dimethyl-propyl)-4-iodo-benzene of formula (VIa) 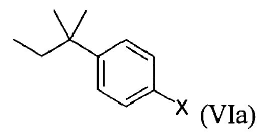 said compound being useful in the process of claim 1,
**characterized in that** it is prepared by reacting a (1,1-dimethyl-propyl)-benzene of formula (VIII) 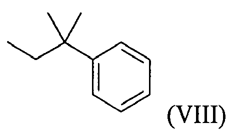 with sodium metaperiodate and iodine in a mixture of acetic acid and acetic anhydride followed by addition of sulfuric acid.
